(19) Europäisches Patentamt
European Patent Office
Office européen des brevets

(11) **EP 3 757 575 B1**

(12) **EUROPEAN PATENT SPECIFICATION**

(45) Date of publication and mention
of the grant of the patent:
**07.06.2023 Bulletin 2023/23**

(21) Application number: **19181942.4**

(22) Date of filing: **24.06.2019**

(51) International Patent Classification (IPC):
*G01N 35/00* ^(2006.01)    *G16H 10/40* ^(2018.01)

(52) Cooperative Patent Classification (CPC):
**G01N 35/00623; G01N 35/0092; G16H 10/40;**
G01N 2035/00633; G01N 2035/0094

(54) **METHOD OF OPERATING AN ANALYTICAL LABORATORY**

VERFAHREN ZUM BETRIEB EINES ANALYSELABORS

PROCÉDÉ DE FONCTIONNEMENT D'UN LABORATOIRE ANALYTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**30.12.2020 Bulletin 2020/53**

(73) Proprietor: **F. Hoffmann-La Roche AG**
**4070 Basel (CH)**

(72) Inventors:
• **MAETZLER, Marco**
**6343 Rotkreuz (CH)**
• **SINZ, Achim**
**70374 Stuttgart (DE)**

(74) Representative: **Ko, Benjamin Hyensuk et al**
**Roche Diagnostics GmbH**
**Department Roche Diagnostics International AG**
**Sandhofer Strasse 116**
**68305 Mannheim (DE)**

(56) References cited:
**CA-A1- 3 046 815    US-A1- 2010 250 174**

**Description**

TECHNICAL FIELD

The present application relates to a computer implemented method of operating an analytical laboratory, in particular an in-vitro diagnostic laboratory. The application further relates to an analytical laboratory configured to carry out the disclosed method. The application further relates to a computer program product comprising instructions which, when executed by a control unit of an analytical laboratory causes it to carry out the disclosed method.

BACKGROUND

In vitro diagnostic testing has a major effect on clinical decisions, providing physicians with pivotal information. In analytical laboratories, in particular, in-vitro diagnostic laboratories, a multitude of analyses on biological samples are executed by laboratory instruments in order to determine physiological and biochemical states of patients, which can be indicative of a disease, nutrition habits, drug effectiveness, organ function and the like.

According to established laboratory procedures in complex analytical laboratories, a plurality of instruments process biological samples according to test orders, each test order defining one or more processing steps to be carried out on the biological sample. After the biological sample has been received and identified by a pre-analytical laboratory instrument, a control unit retrieves the corresponding test orders and determines which instruments are required to process the biological sample according to the test order(s). Having identified the required instrument(s), the control unit determines a sample workflow for each sample according to the test order(s). The sample workflow comprising a sequence and/or timing of carrying out the one or more test orders by the one or more analytical instruments.

In current laboratories biological samples are processed, transported and sometimes even stored at room temperature. Once all test orders related to the biological sample are completed, the sample is stored in an archive (usually refrigerated) or discarded.

However, it has been observed that certain analytes and biological samples degrade over time, in particular if stored at room temperature. Therefore, the validity of certain analytical tests can no longer be guaranteed after the sample has been stored beyond a certain period of time, hereafter referred to as degradation limit.

Known solutions exist which track the temperature of an entire analytical laboratory and/or an analytical instrument, raising an alarm or flagging test results if a critical temperature is exceeded. The disadvantage of such a solution is that it can only address a general problem applicable to all samples in the laboratory/ analytical instrument. However, sample degradation does not only occur due to exceeding a critical temperature but also at normal operating temperature of an analytical laboratory.

EP2339353 describes an analytical laboratory which is configured to determine whether in view of the storage conditions, analysis results of the respective biological sample are valid. While the system disclosed in EP2339353 is configured to identify invalid analysis results, the system is reactive to biological samples with exceeded degradation limits. With such a reactive system, a substitute biological sample needs to be provided for each sample with an exceeded degradation limit. However, providing a substitute biological sample often leads to delays in the total turn around time (the time by which the analysis result becomes available) and/or inconvenience to the patient since biological sample needs to be collected again. Furthermore, in case of new born babies or in forensics, providing a substitute biological sample is not even possible.

Hence there is a need for a method of operating an analytical laboratory, respectively an analytical laboratory system which can proactively prevent that the degradation limit of biological sample(s) is exceeded.

SUMMARY

Embodiments disclosed herein address the need for a method of operating an analytical laboratory, respectively an analytical laboratory system which can proactively prevent that the degradation limit of biological sample(s) is exceeded, by determining/re-determining the sample workflows for test orders in an analytical laboratory taking into account a projected lead time of the biological samples, prioritizing test orders of biological samples which otherwise would exceed the degradation limit.

The disclosed method of operating an analytical laboratory, comprises the steps of:

- Receiving and identifying a plurality of biological samples.

- Upon identifying the biological samples, recording data (e.g. a timestamp) indicative of a time at which the biological samples have been first identified. According to embodiments disclosed, the first identification - and hence the timestamp recorded - of the biological samples is performed at collection of the biological samples (e.g. by the phlebotomist) or by a laboratory instrument, in particular a pre-analytical laboratory instrument.

- Retrieving - by a control unit of the analytical laboratory - an order list from a data storage unit comprising one or more test orders corresponding to each biological sample. A test order defines one or more processing steps to determine presence; absence and/or concentration of an analyte in the biological sample.

- Retrieving from the data storage unit - by the control unit - data indicative of a degradation limit corresponding to each test order of the order list.

The degradation limit is indicative of a maximum time a biological sample may be stored after which validity of the test order would no longer be guaranteed. According to embodiments disclosed herein, the degradation limit comprises multiple time limits, each associated with a particular storage condition (e.g. temperature/ humidity ranges, capped/uncapped sample containers).

- Determining - by the control unit - sample workflows corresponding to each biological sample and the order list.

The sample workflow comprises a sequence and/or timing of carrying out the one or more test orders by analytical instruments of the analytical laboratory.

- Instructing - by the control unit - one or more analytical instruments of the analytical laboratory to carry out the test orders according to the sample workflow.

According to the disclosed method, the step of determining the sample workflows comprises the following substeps:

i) Determining a target list of one or more analytical instruments of the analytical laboratory capable of carrying out the one or more test orders. According to embodiments disclosed herein, the target list of analytical instruments is determined based on the availability and capability of the analytical instruments to process the biological samples according to the corresponding test orders.

ii) Determine a sequence and/or timing of carrying out the one or more test orders by the target list of analytical instruments. According to embodiments disclosed herein, sequence and/or timing of carrying out the one or more test orders is determined by the control unit using a set of rules taking in consideration aspects comprising (but not limited to) decontamination level of each instrument; load balancing between instruments; and/or urgency of test orders.

iii) Calculating an estimated completion time for each test order based on the sequence and/or timing of carrying out the one or more test orders. According to embodiments disclosed herein, the completion time is calculated based on estimated duration of each processing step required to complete the respective test order, the duration being estimated based on manufacturer specifications; statistical values; and/or estimated based on current processing capacity/speed of the respective laboratory instruments.
According to various embodiments disclosed herein, the estimated completion time further comprises a transportation time to the respective laboratory instruments and/or a reserve time accounting for unforeseen variations of the actual processing time of the biological samples as compared to the estimated duration of each processing step.

iv) Determining a lead time corresponding to each biological sample for each test order.
The lead time is the time period between the time at which the biological sample has first been identified and the estimated completion time of the respective test order.

v) Prioritizing one or more test orders from the order list if the lead time exceeds the degradation limit corresponding to the respective test order. According to embodiments disclosed herein, the step of prioritizing one or more test orders from the order list comprises adjusting the sequence and/or timing of carrying out the one or more test order such that the test order with a lead time exceeding the degradation limit is carried out at an earlier time as compared to the sample workflow before the prioritization.

The steps ii) to v) are repeated until the lead time doesn't exceed the degradation limit for any of the test orders or until the steps ii) to v) have been repeated for a number N of iterations.
The method respectively the system disclosed herein are advantageous as they proactively estimate the lead time of each biological sample and in N iterations prioritize the processing of biological samples which would exceed their degradation limit. In such a way the waste of biological samples and/or the inconvenience caused to patient(s) are reduced and/or sample result turnaround time(s) is greatly improved.

BRIEF DESCRIPTION OF THE DRAWINGS

Further characteristics and advantages of the disclosed method / device / system will in the following be described in detail by means of the description and by making reference to the following drawings:

Fig. 1 A highly schematic block diagram of an embodiment of the disclosed analytical laboratory;

Fig. 2 A flowchart illustrating a first embodiment of the disclosed method;

Fig. 3 A flowchart illustrating further embodiments of the disclosed method;

Fig. 4 A flowchart illustrating further embodiments of the disclosed method;

Fig. 5 A flowchart illustrating further embodiments of the disclosed method;

Fig. 6 A highly schematic block diagram of an embodiment of a pre-analytical laboratory instrument of the disclosed analytical laboratory;

Fig. 7 A highly schematic block diagram of a further embodiment of a pre-analytical laboratory instrument of the disclosed analytical laboratory;

Fig. 8 A highly schematic block diagram of an embodiment of an analytical laboratory instrument of the disclosed analytical laboratory;

Fig. 9 A highly schematic block diagram of an embodiment of a post-analytical laboratory instrument of the disclosed analytical laboratory.

DETAILED DESCRIPTION

The use of the 'a' or 'an' are employed to describe elements and components of the embodiments herein. This is done merely for convenience and to give a general sense of the inventive concepts. This description should be read to include one or at least one and the singular also includes the plural unless it is obvious that it is meant otherwise.

As used herein qualifiers such as 'about,' 'approximately,' and 'substantially' are intended to signify that the item or value being qualified is not limited to the exact value or amount specified, but includes some slight variations or deviations therefrom, caused by measuring error or imprecision, manufacturing tolerances, stress exerted on various parts, wear and tear, and combinations thereof, for example.

The terms 'sample', 'patient sample' and 'biological sample' refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term 'analyte' is a component of a sample to be analyzed, e.g. molecules of various sizes, ions, proteins, metabolites, nucleic acid sequences and the like. Information gathered on an analyte may be used to evaluate the impact of the administration of drugs on the organism or on particular tissues or to make a diagnosis. Thus 'analyte' is a general term for substances for which information about presence; absence and/or concentration is intended. Examples of analytes are e.g. glucose, coagulation parameters, endogenic proteins (e.g. proteins released from the heart muscle), metabolites, nucleic acids and so on.

The term 'laboratory instrument' as used herein encompasses any apparatus or apparatus component operable to execute and/or cause the execution of one or more processing steps / workflow steps on one or more biological samples and/or one or more reagents. The expression 'processing steps' thereby refers to physically executed processing steps such as centrifugation, aliquotation, sample analysis and the like. The term 'instrument' covers pre-analytical instruments, post-analytical instruments, analytical instruments and laboratory middleware.

The term 'post-analytical instrument' as used herein encompasses any apparatus or apparatus component that is configured to perform one or more post-analytical processing steps / workflow steps comprising - but not limited to - sample unloading, transport, recapping, decapping, temporary storage/ buffering, archiving (refrigerated or not), retrieval and/ or disposal.

The term 'pre-analytical instrument' as used herein encompasses any apparatus or apparatus component that is configured to perform one or more pre-analytical processing steps / workflow steps comprising - but not limited to - centrifugation, resuspension (e.g. by mixing or vortexing), capping, decapping, recapping, sorting, tube type identification, sample quality determination and/or aliquotation steps. Said processing steps may also comprise adding chemicals or buffers to a sample, concentrating a sample, incubating a sample, and the like.

The term 'analyzer' / 'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'laboratory middleware' as used herein refers to any physical or virtual processing device configurable to control a laboratory instrument / or system comprising one or more laboratory instruments in a way that workflow(s) and workflow step(s) are conducted by the laboratory instrument / system. The laboratory middleware may, for example, instruct the laboratory instrument / system to conduct pre-analytical, post analytical and analytical workflow(s)/ workflow step(s). The laboratory middleware may receive information from a data management unit regarding which steps need to be performed with a certain sample. In some embodiments, the laboratory middleware is integral with a data management unit, is comprised by a server computer and/or be part of one laboratory instrument or even distributed across multiple instruments of the analytical laboratory. The laboratory middleware may, for instance, be embodied as a programmable logic controller running a computer-readable program provided with instructions to perform operations.

The term 'sample transportation system' as used herein encompasses any apparatus or apparatus component that is configured to transport sample carriers (each holding one or more sample containers) between laboratory instruments. In particular, the sample transportation system is a one dimensional conveyor-belt based system, a two-dimensional transportation system (such as a magnetic sample carrier transport system) or a combination thereof.

An 'analytical laboratory' as used herein comprises a system comprising one or more analytical; pre- and post-analytical laboratory instruments; a sample transportation system and/or a laboratory middleware.

The term 'analysis or 'analytical test' as used herein encompasses a laboratory procedure characterizing a parameter of a biological sample for qualitatively assessing or quantitatively measuring the presence or amount or the functional activity of an analyte.

The 'term consumable' comprises - but is not limited - to reagents, system fluids, quality control material, calibrator materials, microplates/ microwell plates, reaction vessels, measurement cuvettes, sample tubes, pipetting tips, etc.

As used herein, the term "calibrator" refers to a composition containing a known concentration of an analyte for use in determining the concentration of the analyte in a sample containing an unknown concentration of the analyte.

The term 'communication network' as used herein encompasses any type of wireless network, such as a WiFi™, GSM™, UMTS or other wireless digital network or a cable based network, such as Ethernet™ or the like. In particular, the communication network can implement the Internet protocol (IP). For example, the communication network comprises a combination of cable-based and wireless networks.

The term 'remote system' or 'server' as used herein encompasses any physical machine or virtual machine having a physical or virtual processor, capable of receiving; processing and sending data. A server can run on any computer including dedicated computers, which individually are also often referred to as 'the server' or shared resources such as virtual servers. In many cases, a computer can provide several services and have several servers running. Therefore, the term server shall encompass any computerized device that shares a resource with one or more client processes. Furthermore, the terms 'remote system' or 'server' encompasses a data transmission and processing system distributed over a data network (such as a cloud environment).

The term 'user interface' as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

The term 'Quality control' or 'analytical quality control' refers to all those processes and procedures designed to ensure that the results of laboratory analysis (analytical tests) are consistent, comparable, accurate and within specified limits of precision.

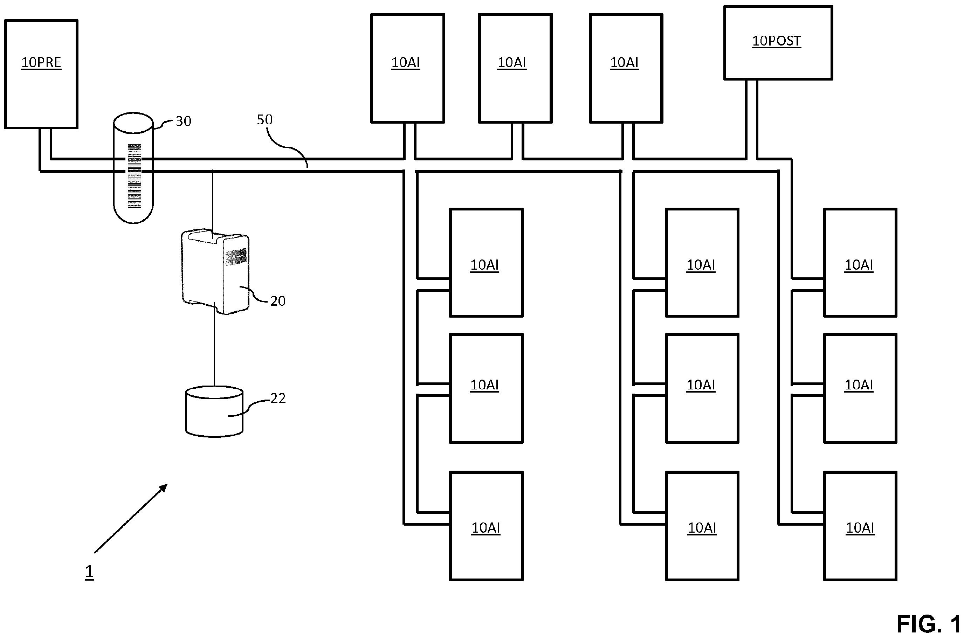

Fig. 1 shows a highly schematic block diagram of an embodiment of the disclosed analytical laboratory 1. As shown on the block diagram of figure 1, embodiments of the disclosed analytical laboratory 1 for processing biological sample(s) comprise a plurality of laboratory instruments 10AI, 10PRE, 10POST and a control unit 20 communicatively connected by a communication network. The plurality of laboratory instruments 10AI, 10PRE, 10POST are configured to execute processing steps on the biological samples according to instructions from the control unit 20.

The pre-analytical instruments 10PRE comprised by the analytical laboratory 1 may be one or more from the list comprising: an instrument for centrifugation of samples, a capping-, decapping- or recapping instrument, aliquoter, a buffer to temporarily store biological samples or aliquots thereof.

The post-analytical instruments 10POST comprised by the analytical laboratory 1 may be one or more from the list comprising: a recapper, an unloader for unloading a sample from an analytical system and/or transporting the sample to a data storage unit or to a unit for collecting biological waste.

According to various embodiments of the disclosed analytical laboratory 1, the plurality of laboratory instruments 10AI, 10PRE, 10POST may be identical or different instruments such as clinical- & immunochemistry analyzers, coagulation chemistry analyzers, immunochemistry analyzers, urine analyzers, nucleic acid analyzers, hematology instruments etc.

The control unit 20 is configured to control the analytical laboratory 1 to carry out the steps of one or more of the methods herein disclosed and is communicatively connected to the data storage unit 22.

As shown on figure 1, the analytical laboratory 1 further comprises a sample transportation system 50 interconnecting the plurality of laboratory instruments 10AI, 10PRE, 10POST. According to embodiments disclosed herein, the sample transportation system 50 is a one dimensional conveyor-belt based system. According to further embodiments disclosed (but not illustrated) the sample transportation system 50 is a two-dimensional transportation system (such as a magnetic sample carrier transport system). The analytical laboratory 1 is configured to carry out the method according to the embodiments disclosed herein.

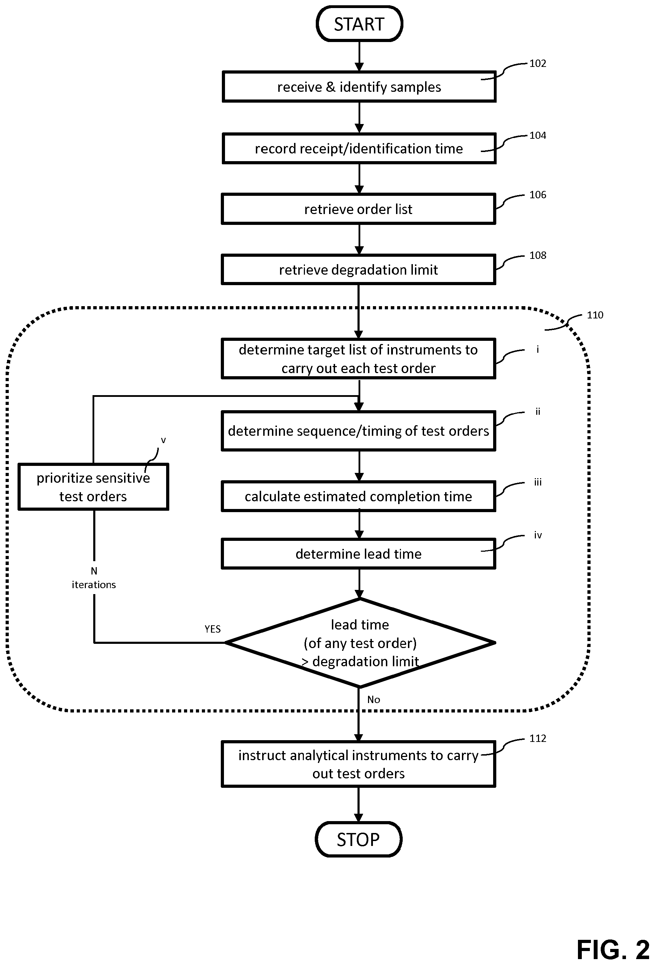

Turing now to figures 2 to 5, embodiments of the disclosed method of operating an analytical laboratory shall be described with reference to the figures.

As shown on figure 2, in a first step 102 of the claimed method, samples are received and identified. According to embodiments disclosed herein, the receipt and identification of samples is performed by a phlebotomist collecting the biological sample(s), a laboratory technician handling the biological sample(s) and/or the first laboratory instrument 10AI, 10PRE, 10POST of the analytical laboratory 1 that the biological sample(s) is loaded onto, in particular a pre-analytical laboratory instrument 10PRE. Thereafter, in a step 104, upon identifying the biological samples, data is recorded indicative of a time at which the biological samples have been first identified. Analogous to the embodiments mentioned above, the data indicative of a time at which the biological samples have been first identified is recorded by the phlebotomist collecting the biological sample(s), a laboratory technician handling the biological sample(s), the control unit 20 being configured for receiving input indicative of a time at which the biological samples have been collected, for example via a mobile app, a graphical user interface or a speech recognition system directly or indirectly communicatively connected to the control unit 20. Alternatively, or additionally, the data indicative of a time at which the biological samples have been first identified is recorded by the one or more laboratory instrument(s) 10PRE, 10AI, 10POST configured to receive and identify biological sample(s).

In order to determine which processing steps need to be carried out on each biological sample, in a step 106, the control unit 20 retrieves an order list from a data storage unit 22 comprising one or more test orders corresponding to each biological sample. A test order defines one or more processing steps to determine presence; absence and/or concentration of an analyte in the biological sample.

To determine the sensitivity of a test order to sample degradation (due to extended storage), in a step 108, data indicative of a degradation limit corresponding to each test order of the order list is retrieved by the by the control unit 20 from the data storage unit 22. The degradation limit is indicative of a maximum time a biological sample may be stored after which validity of the test order could no longer be guaranteed. According to embodiments disclosed herein, the degradation limit comprises multiple time limits, each associated with a particular storage condition (e.g. temperature/ humidity ranges, capped/uncapped sample containers).

Thereafter, in a step 110, the control unit 20 determines sample workflows for each biological sample. A sample workflow comprises a sequence and/or timing of carrying out the one or more test orders by analytical instruments of the analytical laboratory 1. Furthermore, according to embodiments disclosed herein the sample workflow further comprises instructions which, when executed by the respective laboratory instruments, cause the laboratory instruments 10AI, 10PRE, 10POST to carry out the processing steps as defined by the test orders.

As shown on figure 1 as well, the step 110 of determining sample workflows for each biological sample comprises several substeps. In a substep 110 i), the control unit 20 determines a target list of one or more analytical instruments 10AI of the analytical laboratory 1 for carrying out the one or more test orders. According to embodiments disclosed herein, the target list of analytical instruments 10AI is determined based on the availability and capability thereof to process the biological samples according to the corresponding test orders. According to particular embodiments disclosed herein, determining a target list of one or more analytical instruments 10AI of the analytical laboratory 1 capable of carrying out the one or more test orders comprises the steps of: determining whether the laboratory instrument 10AI, 10PRE, 10POST is powered on and not in a low-power mode; determining whether all modules of the laboratory instrument 10AI, 10PRE, 10POST required to carry out the respective test order are operational; determining whether all consumables required to carry out the respective test order are available; and/or determining whether all quality control and/or calibration values of the laboratory instrument 10AI, 10PRE, 10POST are up-to-date and valid.

In a substep 110 ii) the control unit 20 determines a sequence and/or timing of carrying out the one or more test orders by the one or more analytical instruments 10AI of the target list. According to embodiments disclosed herein, the sequence and/or timing of carrying out the one or more test orders is determined by the control unit 20 using a set of rules (also called a rule engine) taking in consideration aspects comprising (but not limited to) decontamination level of each instrument 10AI (a highly decontaminated instrument should receive biological samples before any instrument of lower decontamination level); load balancing between instruments; and/or urgency of test orders and/or a multitude of laboratory specific configuration rules.

In substep 110 iii), the control unit 20 calculates an estimated completion time for each test order based on the sequence and/or timing of carrying out the one or more test orders. According to embodiments disclosed herein, the estimated completion time for each test order is determined based on estimated duration of each processing step of each test order preceding the current test order for the respective laboratory instruments 10AI, 10PRE, 10POST and the estimated duration of each processing step of the current test order. In other words, the estimated completion time takes into consideration all processing steps that need to be carried out before the current test order and the processing steps of the current test order. This estimation is based on historical data and/or instrument specification(s) and/or a setting by a laboratory technician and/or estimated based on current processing capacity/speed of the respective laboratory instruments. According to embodiments disclosed herein, the estimated duration of each processing step is (re)adjusted based on measured duration of the processing step(s).

According to further embodiments disclosed herein, the estimated completion time further comprises a transportation time to the respective laboratory instruments 10AI, 10PRE, 10POST and/or a reserve time accounting for unforeseen variations of the actual processing time of the biological samples as compared to the estimated duration of each processing step.

Having determined the estimated completion time, in a subsequent substep 110 iv), the control unit 20 determines a lead time corresponding to each biological sample for each test order. The lead time as used herein refers to the time period between the time at which the biological sample has first been identified and the estimated completion time of the respective test order.

If the lead time exceeds the degradation limit corresponding to a test order, in substep 110 v), the control unit 20 prioritizes the respective test orders from the order list. In other words, if the control unit 20 estimates that by the time the biological sample would be processed according to the test order, the sample would be degraded, the particular test order is prioritized. According to embodiments disclosed herein, the step 110 v) of prioritizing one or more test orders from the order list comprises adjusting the sequence and/or timing of carrying out the one or more test orders such that the test order with a lead time exceeding the degradation limit is carried out at an earlier time as compared to the sample workflow before the prioritization. It shall be noted that depending on the particular test orders of the analytical laboratory 1, the prioritization may or may not affect test orders of other biological samples.

In order to ensure that the prioritization reached its objective and did not negatively affect other test orders, steps 110 ii) to v) are repeated until the lead time doesn't exceed the degradation limit for any of the test orders. Since there are occasions when, despite (re) prioritization of test orders, a set of sample workflows cannot be determined where the lead time doesn't exceed the degradation limit for any of the test orders, steps 110 ii) to v) are repeated for a number N of iterations.

Once the lead time doesn't exceed the degradation limit for any of the test orders or once steps ii) to v) have been repeated for a number N of iterations, in a step 112, the control unit 20 instructs the one or more analytical instruments 10AI of the analytical laboratory to carry out the test orders according to the sample workflows.

According to embodiments disclosed herein, the control unit 20 associates a processing priority level with each test order and - within the step of prioritizing one or more test orders from the order list - increases the processing priority level for each test order with a lead time exceeding the degradation limit. Correspondingly, the step 112 comprises instructing - by the control unit 20 - one or more analytical instruments 10AI of the analytical laboratory to carry out the test orders according to the respective processing priority level.

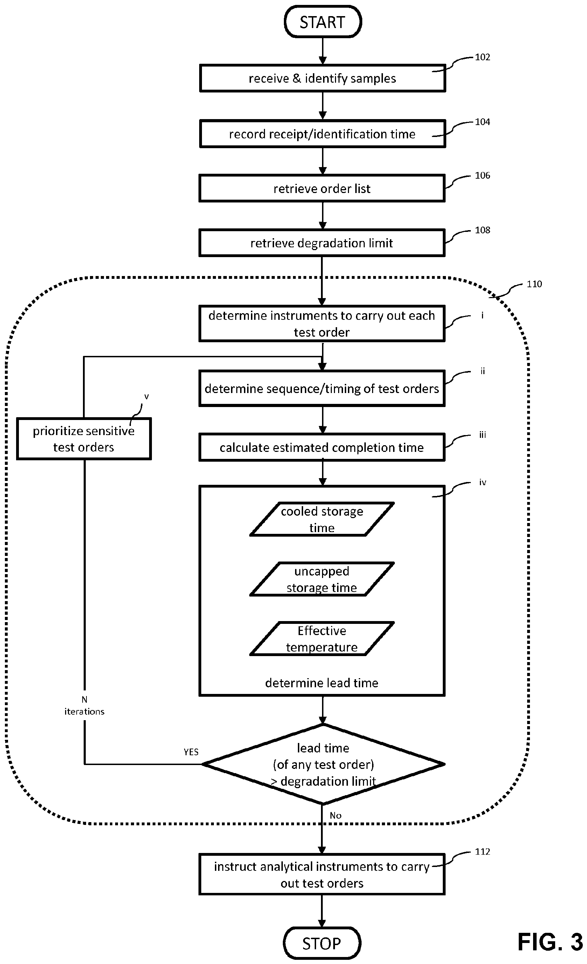

Fig. 3 is a flowchart illustrating further embodiments of the disclosed method, showing further details on the determination of the lead time corresponding to test orders. As illustrated on figure 3, further embodiments disclosed herein further comprise the steps of:

- Registering a cooled storage time period during which each biological sample has been stored cooled by one or more laboratory instruments 10PRE, 10AI or 10POST comprising a refrigerated area for storing the biological sample. The registration of the cooled storage time is based on timestamps provided by the respective laboratory instrument 10PRE, 10AI or 10POST, timestamps at which the respective biological sample was loaded respectively unloaded from the refrigerated area. Furthermore, the respective laboratory instrument 10PRE, 10AI or 10POST may provide data indicative of the actual temperature within the refrigerated area. Alternatively, or additionally, the respective laboratory instrument 10PRE, 10AI or 10POST may provide data confirming that the refrigerated area complies with cooled storage requirements (set either by a lab operator, a regulatory body or manufacture(s) of analytical tests).

- In determining the lead time corresponding to the biological samples, accounting for the cooled storage time period multiplied by a cooled degradation factor. Since biological samples degrade at a different rate when cooled, the time spent by the biological sample(s) in a refrigerated area is multiplied by a factor representative of the different rate of degradation when cooled. Most biological samples degrade at a slower rate when cooled, therefore the cooled degradation factor is less than 1 for such samples.

As illustrated on figure 3, further embodiments disclosed herein further comprise the steps of:

- Registering an uncapped storage time of each biological sample, as a time the biological sample has been in the analytical laboratory 1 uncapped. The registration of the uncapped storage time is based on timestamps provided by the respective laboratory instrument 10PRE, 10AI or 10POST, timestamps at which the respective biological sample was uncapped respectively recapped.

- In determining the lead time corresponding to the biological samples, accounting for the uncapped storage time multiplied by an uncapped degradation factor. Since biological samples degrade at a different rate when the cap is removed (exposure to ambient air) from the sample container holding the biological sample, the time spent by the biological sample(s) uncapped is multiplied by a factor representative of the different rate of degradation when uncapped. Most biological samples degrade at a faster rate when uncapped, therefore the uncapped degradation factor is greater than 1 for such samples.

As illustrated on figure 3, further embodiments disclosed herein further comprise the steps of:

- The control unit 20 receiving data indicative of an effective temperature of storage of the biological samples from one or more of the laboratory instruments 10PRE, 10AI, 10POST.

   Alternatively, or additionally, the control unit 20 receives data from one or more temperature sensors located within the analytical laboratory 1 indicative of an effective temperature of storage of the biological samples, in particular ambient temperature.
   Alternatively, or additionally, the control unit 20 receives data from temperature sensitive label(s) (passive or active e.g. RFID) attached to sample containers holding the biological samples, the temperature sensitive label(s) recording data indicative of average, maximum and/or minimum storage temperature of the biological sample.

- Correspondingly, the lead time corresponding to the biological samples is determined as a function of the corresponding effective temperature. For example, the function could be an integral of the temperature curve corresponding to the biological sample. As a further example, the function may return a maximum value of a critical storage temperature has been exceeded.

According to further embodiments disclosed herein the lead time corresponding to the biological samples is determined as a function of the corresponding cooled storage time; uncapped storage time; and/or effective temperature, such as a weighted average function. For example,

$$\text{storage time} = \quad 1x \text{ storage time at room temperature DE-CAPPED} +$$

$$0.5x \text{ storage time at room temperature CAPPED} +$$

$$0.1x \text{ storage time in fridge} +$$

$$4x \text{ storage time above room temperature DE-CAPPED} +$$

$$2X \text{ storage time above room temperature CAPPED}$$

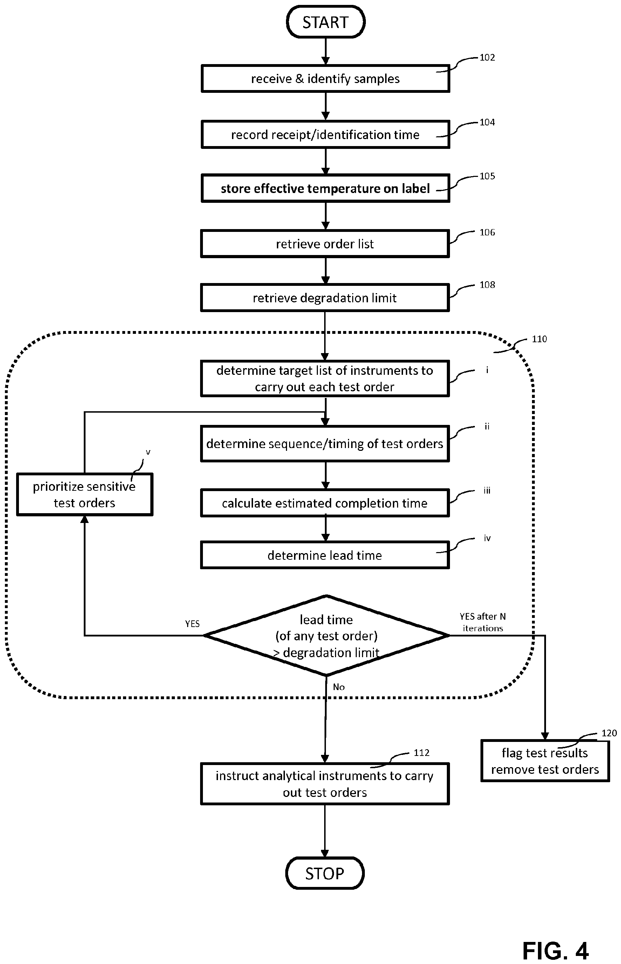

Figure 4 shows a flowchart illustrating further embodiments of the disclosed method, wherein, in a step 120 test orders with a lead time exceeding the degradation limit are flagged by the control unit 20, said flag comprising data indicative of the lead time. The flagged results may then be reviewed by an expert and/or discarded. Alternatively, or additionally, the control unit 20 removes test orders from the sample workflow with a lead time exceeding the degradation limit. According to particular embodiments disclosed herein, the control unit 20 is configured for receiving a manual override approving one or more sample workflow(s) despite the corresponding lead time(s) exceeding the degradation limit(s) for one or more of the test orders. In such a way an operator has the option to carry out the test orders despite the degradation limit(s) exceeded.

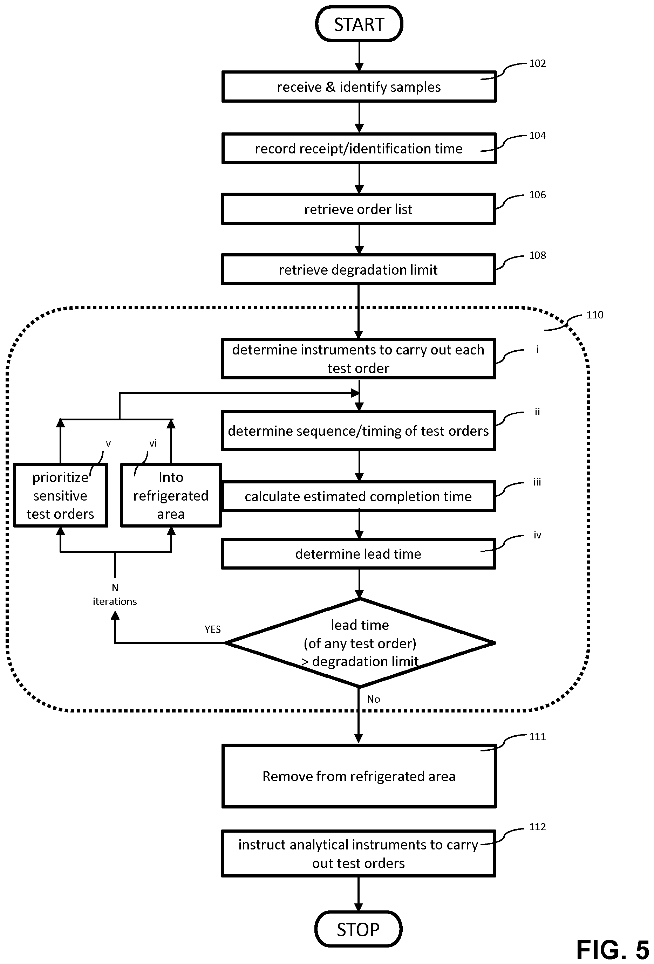

Figure 5 shows a flowchart illustrating further embodiments of the disclosed method for an analytical laboratory 1 further comprising a sample transportation system 50 configured to transport biological samples between laboratory instrument 10PRE, 10AI or 10POST. As shown on figure 5, in a substep 110 vi), the control unit 20 instructs a sample transportation system 50 of the analytical laboratory 1 to transport the biological sample into a laboratory instruments 10PRE, 10AI or 10POST comprising a refrigerated area, if the lead time exceeds the degradation limit for any of the test orders. Thereafter, having recalculated the lead time (based on cooled storage of the biological sample up until processing), in a step 111 the control unit 20 instructs the sample transportation system 50 to transport the biological sample to the laboratory instrument 10AI, 10PRE, 10POST determined to carry out the respective test order if the lead time does not exceed the degradation limit. In such embodiments, the estimated completion time further comprises a transportation time of the biological sample to the laboratory instrument 10AI, 10PRE, 10POST determined to carry out the respective test order.

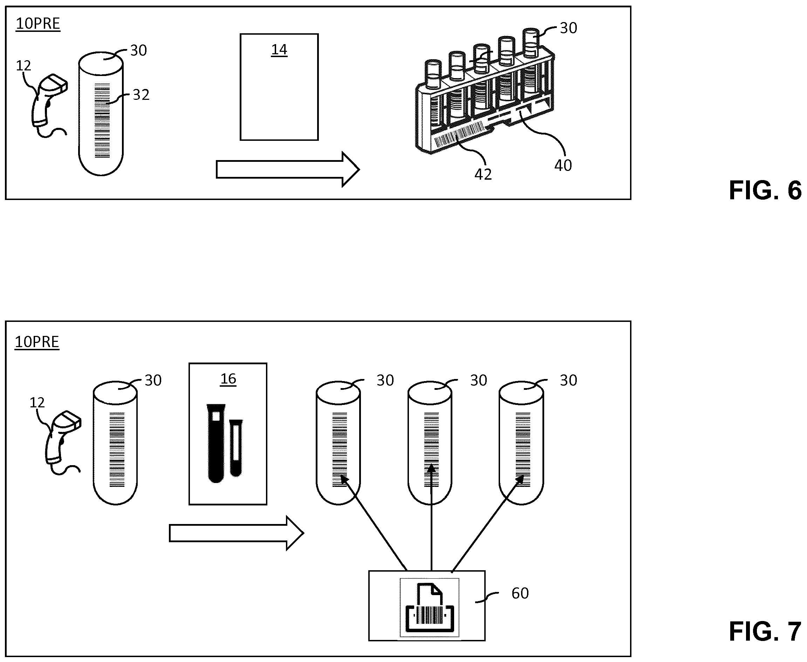

Turning now to figures 6 through 9, particular embodiments of the laboratory instruments 10PRE, 10POST, 10AI are described.

Figure 6 shows a pre-analytical laboratory instrument 10PRE comprising a sample container sorting unit 14 configured to sort sample containers 30 holding biological samples into sample racks 40, each sample rack 40 being identified by means of a rack identifier of a rack tag 42 attached to the sample rack 40, the pre-analytical laboratory instruments 10PRE being further configured to transmit signals to the laboratory control unit associating the sample identifier(s) ID of sorted sample containers 30 with the sample rack identifier(s) of the corresponding sample rack(s) 40. For embodiments where a pre-analytical laboratory instrument 10PRE sorts sample containers 30 into sample racks 40, one or more analytical laboratory instruments are further configured to read the rack identifier Rack-ID from the rack tag 42 and transmit said rack identifier Rack-ID to the laboratory control unit with the test query.

Figure 7 shows a further embodiment of a pre-analytical laboratory instrument 10PRE, comprising an aliquoting unit 16 configured to prepare aliquots of biological sample(s) from the sample container(s) 30 and provide each of said aliquots with a sample identifier ID on an identifier tag 32 by means of an identifier tag writer 60.

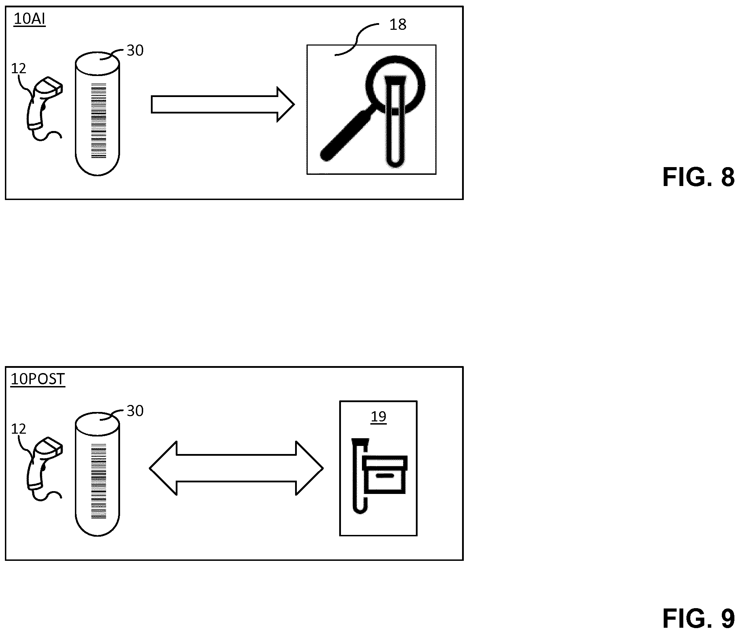

Figure 8 shows an embodiment of an analytical laboratory instrument 10AI, comprising an analytical unit 18 configured to carry out an analytical test to measure the presence, absence and/or concentration of at least one analyte in the biological sample. The analytical laboratory instrument 10AI performs analytical test(s) of the biological sample in response to the test order(s).

Figure 9 shows an embodiment of a post-analytical laboratory instrument 10POST comprising a sample storage unit 19. The post-analytical laboratory instrument 10AI is configured to store respectively retrieve sample containers 30 into respectively from the sample storage unit 19. The query by post-analytical laboratory instrument(s) 10POST to the laboratory control unit for a processing order comprises a container to store respectively retrieve into respectively from the sample storage unit 19. Correspondingly, when queried by a post-analytical laboratory instrument 10POST, the control unit transmits data indicative of a sample container 30 to be retrieved from the sample storage unit 19. In response to the data indicative of a sample container 30 to be stored respectively retrieved, the post-analytical laboratory instrument 10POST stores respectively retrieves the sample container 30 from the sample storage unit 19.

Further disclosed is a computer program product comprising instructions which, when executed by a control unit 20 of an analytical laboratory 1, cause the analytical laboratory 1 to perform the steps of any one of the methods disclosed herein. Thus, specifically, one, more than one or even all of method steps as disclosed herein may be performed by using a computer or a computer network (such as a cloud computing service) or any suitable data processing equipment. As used herein, a computer program product refers to the program as a tradable product. The product may generally exist in any format, such as in a downloadable file, on a computer-readable data carrier on premise or located at a remote location (cloud). The computer program product may be stored on a non-transitory computer-readable data carrier; a server computer as well as on transitory computer-readable data carrier such as a data carrier signal. Specifically, the computer program product may be distributed over a data network. Furthermore, not only the computer program product, but also the execution hardware may be located on-premise or in a remotely, such as in a cloud environment.

Further disclosed and proposed is a non-transitory computer-readable storage medium comprising instructions which, when executed by a control unit 20 of an analytical laboratory 1, cause the analytical laboratory 1 to perform the steps of any one of the methods disclosed herein.

Further disclosed and proposed is a modulated data signal comprising instructions which, when executed by a control unit 20 of an analytical laboratory 1, cause the analytical laboratory 1 to perform the steps of any one of the methods disclosed herein.

REFERENCE LIST:

| | |
|---|---|
| analytical laboratory | 1 |
| laboratory instrument | 10PRE, 10POST, 10AI |
| pre-analytical laboratory instrument | 10PRE |
| analytical laboratory instrument | 10AI |
| post-analytical laboratory instrument | 10POST |
| identifier tag reader | 12 |
| sample container sorting unit | 14 |
| aliquoting unit | 16 |
| analytical unit | 18 |
| sample storage unit | 19 |
| control unit | 20 |
| data storage unit | 22 |
| sample container | 30 |
| identifier tag | 32 |
| sample rack | 40 |
| sample rack tag | 42 |
| sample transportation system | 50 |
| identifier reader unit (of transport system) | 52 |
| receive; identify and samples | step 102 |
| record receipt/identification time | step 104 |
| retrieve order list | step 106 |
| retrieve degradation limit | step 108 |
| determining sample workflows | step 110 |
| determine target list of instruments to carry out each test order | step 110 i) |
| determine sequence/timing of test orders | step 110 ii) |
| determine estimated completion time | step 110 iii) |
| determine lead time | step 110 iv) |
| prioritize sensitive test orders | step 110 v) |
| transport sample into refrigerated area | step 110 vi) |
| Remove sample from refrigerated area | step 111 |
| instruct analytical instruments to carry out test orders | step 112 |
| flag test results and/or remove test orders | step 120 |

**Claims**

1. A computer implemented method of operating an analytical laboratory (1), comprising the steps of:

- receiving and identifying a plurality of biological samples;
- upon identifying the biological samples, recording data indicative of a time at which the biological samples have been first identified;
- retrieving - by a control unit (20) of the analytical laboratory (1) - an order list from a data storage unit (22) comprising one or more test orders corresponding to each biological sample;
- retrieving from the data storage unit (22) - by the control unit (20) - data indicative of a degradation limit corresponding to each test order of the order list;
- determining - by the control unit (20) - sample workflows corresponding to each biological sample and the order list;
- instructing - by the control unit (20) - one or more laboratory instruments (10AI, 10PRE, 10POST) of the analytical laboratory (1) to carry out the test orders according to the sample workflows,

wherein, the step of determining the sample workflows comprises:

i) determining a target list of one or more laboratory instruments (10AI, 10PRE, 10POST) of the analytical laboratory (1) for carrying out the one or more test orders;
ii) determining a sequence and/or timing of carrying out the one or more test orders by the target list of laboratory instruments (10AI, 10PRE, 10POST);
iii) calculating an estimated completion time for each test order based on the sequence and/or timing of carrying out the one or more test orders;
iv) determining a lead time corresponding to each biological sample for each test order; the lead time being the time period between the time at which the biological sample has first been identified and the estimated completion time of the respective test order;
v) prioritizing one or more test orders from the order list if the lead time exceeds the degradation limit corresponding to the respective test order;

repeating steps ii) to v)

- until the lead time doesn't exceed the degradation limit for any of the test orders; or
- until steps ii) to v) have been repeated for a number N of iterations.

2. A method of operating an analytical laboratory (1) according to claim 1, further comprising the steps of:

- registering a cooled storage time period during which each biological sample has been stored cooled by one or more laboratory instruments (10PRE, 10AI or 10POST) comprising a refrigerated area for storing the biological sample; and
- in determining the lead time corresponding to the biological samples, accounting for the cooled storage time period multiplied by a cooled degradation factor.

3. A method of operating an analytical laboratory (1) according to claim 1 or 2, further comprising the steps of:

- registering an uncapped storage time of each biological sample, as a time the biological sample has been in the analytical laboratory (1) uncapped; and
- in determining the lead time corresponding to the biological samples, accounting for the uncapped storage time multiplied by an uncapped degradation factor.

4. A method of operating an analytical laboratory (1) according to any of the claims 1 to 3, further comprising the steps of:

- the control unit (20) receiving data indicative of an effective temperature of storage of the biological samples from one or more of the laboratory instruments (10PRE, 10AI, 10POST) and/or one or more temperature sensors located within the analytical laboratory (1) and/or a temperature sensitive label attached to sample containers holding the biological samples; and
- determining the lead time corresponding to the biological samples as a function of the corresponding effective temperature.

5. A method of operating an analytical laboratory (1) according to claim 4, further comprising the steps of:

- the one or more of the laboratory instruments (10PRE, 10AI, 10POST) and/or the one or more temperature sensors located within the analytical laboratory (1) storing the effective temperature onto a storage label attached to the sample containers holding the biological samples;
- the control unit (20) reading the effective temperature from the storage labels.

6. A method of operating an analytical laboratory (1) according to any of the preceding claims, further comprising the step(s) of:

- the control unit (20) flagging test results of test orders with a lead time exceeding the degradation limit, said flag comprising data indicative of the lead time; and/or
- the control unit (20) removing test orders from the sample workflow with a lead time exceeding the degradation limit.

7. A method of operating an analytical laboratory (1) according to claim 6 further comprising, the control unit (20) receiving a manual override approving one or more sample workflow(s) despite the corresponding lead time(s) exceeding the degradation limit(s) for one or more of the test orders.

8. A method of operating an analytical laboratory (1) according to any of the preceding claims, wherein the step of prioritizing one or more test orders from the order list comprises adjusting - by the control unit (20) - the sequence and/or timing of carrying out the one or more test order(s) such that the test order with a lead time exceeding the degradation limit is carried out at an earlier time as compared to the sample workflows before the prioritization.

9. A method of operating an analytical laboratory (1) according to any of the preceding claims, further comprising:

- associating - by the control unit (20) - a processing priority level with each test order;
- within the step of prioritizing one or more test orders from the order list, increasing the processing priority level for each test order with a lead time exceeding the degradation limit;
- instructing - by the control unit (20) - one or more laboratory instruments (10AI, 10PRE, 10POST) of the analytical laboratory to carry out the test orders according to the respective processing priority level.

10. A method of operating an analytical laboratory (1) according to any of the preceding claims, wherein the data indicative of a time at which the biological samples have been first identified is recorded by:

- the control unit 20 receiving input indicative of a time at which the biological samples have been collected;
- one or more laboratory instrument(s) (10PRE, 10AI, 10POST) configured to receive and identify biological sample(s).

11. A method of operating an analytical laboratory (1) according to any of the preceding claims, wherein determining a target list of one or more laboratory instruments (10AI, 10PRE, 10POST) of the analytical laboratory (1) for carrying out the one or more test orders comprises the steps of:

- determining whether the laboratory instrument (10AI, 10PRE, 10POST) is powered on and not in a low-power mode; and/or
- determining whether all modules of the laboratory instrument (10AI, 10PRE, 10POST) required to carry out the respective test order are operational; and/or
- determining whether all consumables required to carry out the respective test order are available; and/or
- determining whether all quality control and/or calibration values of the laboratory instrument (10AI, 10PRE, 10POST) are up-to-date and valid.

12. A method of operating an analytical laboratory (1) according to any of the preceding claims, further comprising the steps of:

- instructing - by the control unit (20) - a sample transportation system (50) of the analytical laboratory (1) to transport the biological sample into a laboratory instrument (10PRE, 10AI or 10POST) comprising a refrigerated area, if the lead time exceeds the degradation limit for any of the test orders;
- instructing - by the control unit (20) - the sample transportation system (50) to transport the biological sample to the laboratory instrument (10AI, 10PRE, 10POST) determined to carry out the respective test order if the lead time does not exceed the degradation limit

wherein the estimated completion time further comprises a transportation time of the biological sample to the laboratory instrument (10AI, 10PRE, 10POST) determined to carry out the respective test order.

13. An analytical laboratory (1) comprising:

- one or more laboratory instrument(s) (10PRE, 10AI, 10POST) configured to receive and identify biological sample(s);
- one or more analytical instruments (10AI) configured to determine presence; absence and/or concentration of an analyte in the biological sample;
- a control unit (20) communicatively connected to the laboratory instrument(s) (10PRE, 10AI, 10POST),

the analytical laboratory (1) being configured to carry out the method according to one of the claims 1 to 11.

14. An analytical laboratory (1) according to claim 13 further comprising a sample transportation system (50) configured to transport biological sample(s) between the laboratory instruments (10PRE, 10AI, 10POST); wherein the analytical laboratory (1) is configured to carry out the method according to claim 12.

15. A computer program product comprising instructions which, when executed by a control unit (20) of an analytical laboratory (1), cause the analytical laboratory (1) to perform the steps of any one of the methods according to claims 1 through 12.

**Patentansprüche**

1. Computerimplementiertes Verfahren zum Betreiben eines Analyselabors (1), umfassend die folgenden Schritte:

- Empfangen und Identifizieren einer Vielzahl von biologischen Proben;
- nach dem Identifizieren der biologischen Proben, Aufzeichnen von Daten, die einen Zeitpunkt angeben, zu dem die biologischen Proben zum ersten Mal identifiziert worden sind;
- Abrufen einer Auftragsliste aus einer Datenspeichereinheit (22), umfassend einen oder mehrere Testaufträge, die jeder biologischen Probe entsprechen, mittels einer Steuerungseinheit (20) des Analyselabors (1);
- Abrufen von Daten, die eine Zersetzungsgrenze angeben, die jedem Testauftrag der Auftragsliste entspricht, aus der Datenspeichereinheit (22) mittels der Steuerungseinheit (20);
- Bestimmen von Proben-Workflows, die jeder biologischen Probe und der Auftragsliste entsprechen, mittels der Steuerungseinheit (20);
- Anweisen eines oder mehrerer Laborinstrumente (10AI, 10PRE, 10POST) des Analyselabors (1), die Testaufträge gemäß den Proben-Workflows auszuführen, mittels der Steuerungseinheit (20),

wobei der Schritt des Bestimmens der Proben-Workflows Folgendes umfasst:

i) Bestimmen einer Zielliste von einem oder mehreren Laborinstrumenten (10AI, 10PRE, 10POST) des Analyselabors (1) zum Ausführen des einen Testauftrags oder der mehreren Testaufträge;
ii) Bestimmen einer Abfolge und/oder Planung des Ausführens des einen Testauftrags oder der mehreren Testaufträge mittels der Zielliste von Laborinstrumenten (10AI, 10PRE, 10POST);
iii) Berechnen einer geschätzten Endzeit für jeden Testauftrag basierend auf der Abfolge und/oder Planung des Ausführens des einen Testauftrags oder der mehreren Testaufträge;
iv) Bestimmen einer Durchlaufzeit, die jeder biologischen Probe entspricht, für jeden Testauftrag; wobei die Durchlaufzeit die Dauer zwischen dem Zeitpunkt, zu dem die biologische Probe zum ersten Mal identifiziert worden ist, und der geschätzten Endzeit des jeweiligen Testauftrags ist;
v) Priorisieren eines Testauftrags oder mehrerer Testaufträge aus der Auftragsliste, wenn die Durchlaufzeit die Zersetzungsgrenze, die dem jeweiligen Testauftrag entspricht, überschreitet;

Wiederholen der Schritte ii) bis v)

- bis die Durchlaufzeit die Zersetzungsgrenze für keinen der Testaufträge mehr überschreitet; oder

- bis die Schritte ii) bis v) für eine Anzahl N von Wiederholungen wiederholt worden sind.

2. Verfahren zum Betreiben eines Analyselabors (1) nach Anspruch 1, ferner umfassend die folgenden Schritte:

- Registrieren einer Kühlungslagerdauer, während der jede biologische Probe von einem oder mehreren Laborinstrumenten (10PRE, 10AI oder 10POST), die einen gekühlten Bereich zum Lagern der biologischen Probe umfassen, gekühlt gelagert wird; und
- beim Bestimmen der Durchlaufzeit, die den biologischen Proben entspricht, Berücksichtigen der Kühlungslagerdauer, multipliziert mit einem Kühlungszersetzungsfaktor.

3. Verfahren zum Betreiben eines Analyselabors (1) nach Anspruch 1 oder 2, ferner umfassend die folgenden Schritte:

- Registrieren einer Offenlagerzeit jeder biologischen Probe als eine Zeit, für die die biologische Probe in dem Analyselabor (1) geöffnet war; und
- beim Bestimmen der Durchlaufzeit, die den biologischen Proben entspricht, Berücksichtigen der Offenlagerzeit, multipliziert mit einem Offenzersetzungsfaktor.

4. Verfahren zum Betreiben eines Analyselabors (1) nach einem der Ansprüche 1 bis 3, ferner umfassend die folgenden Schritte:

- Empfangen von Daten, die eine effektive Lagertemperatur der biologischen Proben angeben, von einem oder mehreren der Laborinstrumente (10PRE, 10AI, 10POST) und/oder einem oder mehreren Temperatursensoren, die sich innerhalb des Analyselabors (1) befinden, und/oder einem temperaturempfindlichen Etikett, das an Probenbehältern, die die biologischen Proben halten, angebracht ist, mittels der Steuerungseinheit (20); und
- Bestimmen der Durchlaufzeit, die den biologischen Proben entspricht, als eine Funktion der entsprechenden effektiven Temperatur.

5. Verfahren zum Betreiben eines Analyselabors (1) nach Anspruch 4, ferner umfassend die folgenden Schritte:

- Speichern der effektiven Temperatur auf einem Speicheretikett, das an den Probenbehältern, die die biologischen Proben halten, angebracht ist, mittels des einen oder der mehreren Laborinstrumente (10PRE, 10AI, 10POST) und/oder des einen oder der mehreren Temperatursensoren, die sich innerhalb des Analyselabors (1) befinden;
- Auslesen der effektiven Temperatur aus den Speicheretiketten mittels der Steuerungseinheit (20).

6. Verfahren zum Betreiben eines Analyselabors (1) nach einem der vorstehenden Ansprüche, ferner umfassend den/die folgenden Schritt(e):

- Kennzeichnen von Testergebnissen von Testaufträgen mit einer Durchlaufzeit, die die Zersetzungsgrenze überschreitet, mittels der Steuerungseinheit (20), wobei die Kennzeichnung Daten umfasst, die die Durchlaufzeit angeben; und/oder
- Entfernen von Testaufträgen aus dem Proben-Workflow mit einer Durchlaufzeit, die die Zersetzungsgrenze überschreitet, mittels der Steuerungseinheit (20).

7. Verfahren zum Betreiben eines Analyselabors (1) nach Anspruch 6, ferner umfassend, Empfangen eines manuellen Eingriffs, der einen oder mehrere Proben-Workflow(s) genehmigt, ungeachtet der/den entsprechenden Durchlaufzeit(en), die die Zersetzungsgrenze(n) für einen oder mehrere der Testaufträge überschreitet/überschreiten, mittels der Steuerungseinheit (20).

8. Verfahren zum Betreiben eines Analyselabors (1) nach einem der vorstehenden Ansprüche, wobei der Schritt der Priorisierung eines Testauftrags oder mehrerer Testaufträge von der Auftragsliste das Einstellen der Abfolge und/oder Planung des Ausführens des einen Testauftrags oder der mehreren Testaufträge mittels der Steuerungseinheit (20) umfasst, sodass der Testauftrag mit einer Durchlaufzeit, die die Zersetzungsgrenze überschreitet, im Vergleich zu den Proben-Workflows vor der Priorisierung früher ausgeführt wird.

9. Verfahren zum Betreiben eines Analyselabors (1) nach einem der vorstehenden Ansprüche, ferner umfassend:

- Inverbindungbringen einer Bearbeitungsprioritätsstufe mit jedem Testauftrag mittels der Steuerungseinheit (20);
- Erhöhen der Bearbeitungsprioritätsstufe für jeden Testauftrag mit einer Durchlaufzeit, die die Zersetzungsgrenze überschreitet, innerhalb des Schrittes des Priorisierens eines Testauftrags oder mehrerer Testaufträge aus der Auftragsliste;
- Anweisen eines oder mehrerer Laborinstrumente (10AI, 10PRE, 10POST) des Analyselabors, die Testaufträge gemäß der jeweiligen Bearbeitungsprioritätsstufe auszuführen, mittels der Steuerungseinheit (20).

10. Verfahren zum Betreiben eines Analyselabors (1) nach einem der vorstehenden Ansprüche, wobei die Daten, die einen Zeitpunkt angeben, zu dem die biologischen Proben zum ersten Mal identifiziert worden sind, aufgezeichnet werden durch:

- die Steuerungseinheit 20, die eine Eingabe empfängt, die einen Zeitpunkt angibt, zu dem die biologischen Proben genommen worden sind;
- ein oder mehrere Laborinstrument(e) (10PRE, 10AI, 10POST), die dafür ausgelegt sind, biologische Proben zu empfangen und zu identifizieren.

11. Verfahren zum Betreiben eines Analyselabors (1) nach einem der vorstehenden Ansprüche, wobei das Bestimmen einer Zielliste von einem oder mehreren Laborinstrumenten (10AI, 10PRE, 10POST) des Analyselabors (1) zum Ausführen des einen Testauftrags oder der mehreren Testaufträge die folgenden Schritte umfasst:

- Bestimmen, ob das Laborinstrument (10AI, 10PRE, 10POST) eingeschaltet ist und sich nicht in einem Energiesparmodus befindet; und/oder
- Bestimmen, ob alle Module des Laborinstruments (10AI, 10PRE, 10POST), die zur Ausführung des jeweiligen Testauftrags erforderlich sind, betriebsfähig sind; und/oder
- Bestimmen, ob alle Verbrauchsmaterialien, die zur Ausführung des jeweiligen Testauftrags erforderlich sind, verfügbar sind; und/oder
- Bestimmen, ob alle Qualitätskontroll- und/oder Kalibrierungswerte des Laborinstruments (10AI, 10PRE, 10POST) aktuell und gültig sind.

12. Verfahren zum Betreiben eines Analyselabors (1) nach einem der vorstehenden Ansprüche, ferner umfassend die folgenden Schritte:

- Anweisen eines Probentransportsystems (50) des Analyselabors (1), die biologische Probe in ein Laborinstrument (10PRE, 10AI oder 10POST), das einen gekühlten Bereich umfasst, zu transportieren, wenn die Durchlaufzeit die Zersetzungsgrenze für einen der Testaufträge überschreitet, mittels der Steuerungseinheit (20);
- Anweisen des Probentransportsystems (50), die biologische Probe zu dem Laborinstrument (10PRE, 10AI, 10POST), welches zur Ausführung des jeweiligen Testauftrags bestimmt wurde, zu transportieren, wenn die Durchlaufzeit die Zersetzungsgrenze überschreitet, mittels der Steuerungseinheit (20);

wobei die geschätzte Endzeit ferner eine Transportzeit der biologischen Probe zu dem Laborinstrument (10AI, 10PRE, 10POST), welches zur Ausführung des jeweiligen Testauftrags bestimmt wurde, umfasst.

13. Analyselabor (1), umfassend:

- ein oder mehrere Laborinstrument(e) (10PRE, 10AI, 10POST), die dafür ausgelegt sind, (eine) biologische Probe(n) zu empfangen und zu identifizieren;
- ein oder mehrere Analyseinstrument(e) (10AI), die dafür ausgelegt sind, die Gegenwart; Abwesenheit und/oder Konzentration eines Analyten in der biologischen Probe zu bestimmen;
- eine Steuerungseinheit (20), die kommunikationsfähig mit dem/den Laborinstrument(en) (10PRE, 10AI, 10POST) verbunden ist,

wobei das Analyselabor (1) dafür ausgelegt ist, das Verfahren nach einem der Ansprüche 1 bis 11 auszuführen.

14. Analyselabor (1) nach Anspruch 13, ferner umfassend ein Probentransportsystem (50), das dafür ausgelegt ist, (eine) biologische Probe(n) zwischen den Laborinstrumenten (10PRE, 10AI, 10POST) zu transportieren; wobei das Analyselabor (1) dafür ausgelegt ist, das Verfahren nach Anspruch 12 auszuführen.

15. Computerprogrammprodukt, umfassend Anweisungen, die bei Ausführung von einer Steuerungseinheit (20) eines Analyselabors (1) das Analyselabor (1) veranlassen, die Schritte eines der Verfahren nach den Ansprüchen 1 bis 12 auszuführen.

**Revendications**

1. Procédé mis en oeuvre par ordinateur de fonctionnement d'un laboratoire analytique (1), comprenant les étapes de :

   - réception et identification d'une pluralité d'échantillons biologiques ;
   - lors de l'identification des échantillons biologiques, enregistrement des données indiquant un temps auquel les échantillons biologiques ont été identifiés pour la première fois ;
   - récupération, par une unité de commande (20) du laboratoire analytique (1), d'une liste d'ordres en provenance d'une unité de stockage de données (22) comprenant un ou plusieurs ordres de test correspondant à chaque échantillon biologique ;
   - récupération en provenance de l'unité de stockage de données (22), par l'unité de commande (20), de données indiquant une limite de dégradation correspondant à chaque ordre de test de la liste d'ordres ;
   - détermination, par l'unité de commande (20), de flux de travail des échantillons correspondant à chaque échantillon biologique et à la lise d'ordres ;
   - instruction, par l'unité de commande (20), à un ou plusieurs instruments de laboratoire (10AI, 10PRE, 10POST) du laboratoire analytique (1) de réaliser les ordres de test en fonction des flux de travail des échantillons,

   dans lequel, l'étape de détermination des flux de travail des échantillons comprend :

   i) la détermination d'une liste cible d'un ou de plusieurs instruments de laboratoire (10AI, 10PRE, 10POST) du laboratoire analytique (1) pour réaliser le ou les ordres de test ;
   ii) la détermination d'une séquence et/ou d'un calendrier de réalisation du ou des ordres de test par la liste cible d'instruments de laboratoire (10AI, 10PRE, 10POST) ;
   iii) le calcul d'un temps d'achèvement estimé pour chaque ordre de test sur la base de la séquence et/ou du calendrier de réalisation du ou des ordres de test ;
   iv) la détermination d'un temps d'exécution correspondant à chaque échantillon biologique pour chaque ordre de test ; le temps d'exécution étant la période de temps entre le temps auquel l'échantillon biologique a été identifié pour la première fois et le temps d'achèvement estimé de l'ordre de test respectif ;
   v) la hiérarchisation d'un ou de plusieurs ordres de test parmi la liste d'ordres si le temps d'exécution dépasse la limite de dégradation correspondant à l'ordre de test respectif ;

   la répétition des étapes ii) à v)

   - jusqu'à ce que le temps d'exécution ne dépasse pas la limite de dégradation pour l'un quelconque des ordres de test ; ou
   - jusqu'à ce que les étapes ii) à v) aient été répétées pendant un nombre N d'itérations.

2. Procédé de fonctionnement d'un laboratoire analytique (1) selon la revendication 1, comprenant en outre les étapes de :

   - enregistrement d'une période de temps de stockage au froid pendant laquelle chaque échantillon biologique a été stocké au froid par un ou plusieurs instruments de laboratoire (10PRE, 10AI ou 10POST) comprenant une zone réfrigérée pour stocker l'échantillon biologique ; et
   - pour déterminer le temps d'exécution correspondant aux échantillons biologiques, prise en compte de la période de temps de stockage au froid multipliée par un facteur de dégradation au froid.

3. Procédé de fonctionnement d'un laboratoire analytique (1) selon la revendication 1 ou 2, comprenant en outre les étapes de :

   - enregistrement d'un temps de stockage sans bouchon de chaque échantillon biologique, comme un temps pendant lequel l'échantillon biologique a été dans le laboratoire analytique (1) sans bouchon ; et
   - pour déterminer le temps d'exécution correspondant aux échantillons biologiques, prise en compte du temps de stockage sans bouchon multiplié par un facteur de dégradation sans bouchon.

**4.** Procédé de fonctionnement d'un laboratoire analytique (1) selon l'une quelconque des revendications 1 à 3, comprenant en outre les étapes de :

- l'unité de commande (20) recevant des données indiquant une température de stockage effective des échantillons biologiques en provenance d'un ou de plusieurs des instruments de laboratoire (10PRE, 10AI, 10POST) et/ou d'un ou de plusieurs capteurs de température situés au sein du laboratoire analytique (1) et/ou d'une étiquette sensible à la température fixée aux récipients d'échantillons contenant les échantillons biologiques ; et
- détermination du temps d'exécution correspondant aux échantillons biologiques en fonction de la température effective correspondante.

**5.** Procédé de fonctionnement d'un laboratoire analytique (1) selon la revendication 4, comprenant en outre les étapes de :

- le ou les instruments de laboratoire (10PRE, 10AI, 10POST) et/ou le ou les capteurs de température situés au sein du laboratoire analytique (1) stockant la température effective sur une étiquette de stockage fixée aux récipients d'échantillons contenant les échantillons biologiques ;
- l'unité de commande (20) lisant la température effective à partir des étiquettes de stockage.

**6.** Procédé de fonctionnement d'un laboratoire analytique (1) selon l'une quelconque des revendications précédentes, comprenant en outre la ou les étapes de :

- l'unité de commande (20) signalant les résultats de test des ordres de test avec un temps d'exécution dépassant la limite de dégradation, ledit signalement comprenant des données indiquant le temps d'exécution ; et/ou
- l'unité de commande (20) éliminant les ordres de test du flux de travail des échantillons avec un temps d'exécution dépassant la limite de dégradation.

**7.** Procédé de fonctionnement d'un laboratoire analytique (1) selon la revendication 6 comprenant en outre, l'unité de commande (20) recevant une dérogation manuelle approuvant un ou plusieurs flux de travail des échantillons malgré le ou les temps d'exécution correspondants dépassant la ou les limites de dégradation pour un ou plusieurs des ordres de test.

**8.** Procédé de fonctionnement d'un laboratoire analytique (1) selon l'une quelconque des revendications précédentes, dans lequel l'étape de hiérarchisation d'un ou de plusieurs ordres de test parmi la liste d'ordres comprend l'ajustement, par l'unité de commande (20), de la séquence et/ou du calendrier de réalisation du ou des ordres de test de telle sorte que l'ordre de test ayant un temps d'exécution dépassant la limite de dégradation est réalisé plus tôt par comparaison aux flux de tâches de travail des échantillons avant la hiérarchisation.

**9.** Procédé de fonctionnement d'un laboratoire analytique (1) selon l'une quelconque des revendications précédentes, comprenant en outre :

- l'association, par l'unité de commande (20), d'un niveau de priorité de traitement avec chaque ordre de test ;
- au sein de l'étape de hiérarchisation d'un ou de plusieurs ordres de test parmi la liste d'ordres, l'augmentation du niveau de priorité de traitement pour chaque ordre de test ayant un temps d'exécution dépassant la limite de dégradation ;
- l'instruction, par l'unité de commande (20), à un ou plusieurs instruments de laboratoire (10AI, 10PRE, 10POST) du laboratoire analytique de réaliser les ordres de test en fonction du niveau de priorité de traitement respectif.

**10.** Procédé de fonctionnement d'un laboratoire analytique (1) selon l'une quelconque des revendications précédentes, dans lequel les données indiquant un temps auquel les échantillons biologiques ont été identifiés pour la première fois sont enregistrées par :

- l'unité de commande 20 recevant une entrée indiquant un temps auquel les échantillons biologiques ont été collectés ;
- un ou plusieurs instruments de laboratoire (10PRE, 10AI, 10POST) configurés pour recevoir et identifier un ou plusieurs échantillons biologiques.

**11.** Procédé de fonctionnement d'un laboratoire analytique (1) selon l'une quelconque des revendications précédentes, dans lequel la détermination d'une liste cible d'un ou de plusieurs instruments de laboratoire (10AI, 10PRE, 10POST) du laboratoire analytique (1) pour réaliser le ou les ordres de test comprend les étapes de :

- détermination si l'instrument de laboratoire (10AI, 10PRE, 10POST) est sous tension et non dans un mode basse puissance ; et/ou
- détermination si tous les modules de l'instrument de laboratoire (10AI, 10PRE, 10POST) requis pour réaliser l'ordre de test respectif sont opérationnels ; et/ou
- détermination si tous les consommables requis pour réaliser l'ordre de test respectif sont disponibles ; et/ou
- détermination si toutes les valeurs de contrôle qualité et/ou de calibration de l'instrument de laboratoire (10AI, 10PRE, 10POST) sont à jour et valides.

12. Procédé de fonctionnement d'un laboratoire analytique (1) selon l'une quelconque des revendications précédentes, comprenant en outre les étapes de :

- instruction, par l'unité de commande (20), à un système de transport d'échantillon (50) du laboratoire analytique (1) de transporter l'échantillon biologique à l'intérieur d'un instrument de laboratoire (10AI, 10PRE, 10POST) comprenant une zone réfrigérée, si le temps d'exécution dépasse la limite de dégradation pour l'un quelconque des ordres de test ;
- instruction, par l'unité de commande (20), au système de transport d'échantillon (50) de transporter l'échantillon biologique vers l'instrument de laboratoire (10AI, 10PRE, 10POST) déterminé pour réaliser l'ordre de test respectif si le temps d'exécution ne dépasse pas la limite de dégradation

dans lequel le temps d'achèvement estimé comprend en outre un temps de transport de l'échantillon biologique vers l'instrument de laboratoire (10AI, 10PRE, 10POST) déterminé pour réaliser l'ordre de test respectif.

13. Laboratoire analytique (1) comprenant :

- un ou plusieurs instruments de laboratoire (10PRE, 10AI, 10POST) configurés pour recevoir et identifier un ou des échantillons biologiques ;
- un ou plusieurs instruments analytiques (10AI) configurés pour déterminer la présence ; l'absence et/ou la concentration d'un analyte dans l'échantillon biologique ;
- une unité de commande (20) connectée en communication avec le ou les instruments de laboratoire (10PRE, 10AI, 10POST),

le laboratoire analytique (1) étant configuré pour réaliser le procédé selon l'une des revendications 1 à 11.

14. Laboratoire analytique (1) selon la revendication 13 comprenant en outre un système de transport d'échantillon (50) configuré pour transporter le ou les échantillons biologiques entre les instruments de laboratoire (10PRE, 10AI, 10POST) ; dans lequel le laboratoire analytique (1) est configuré pour réaliser le procédé selon la revendication 12.

15. Produit de programme informatique comprenant des instructions qui, lorsqu'elles sont exécutées par une unité de commande (20) d'un laboratoire analytique (1), amènent le laboratoire analytique (1) à effectuer les étapes de l'un quelconque des procédés selon les revendications 1 à 12.

FIG. 1

**FIG. 2**

START

receive & identify samples — 102

record receipt/identification time — 104

retrieve order list — 106

retrieve degradation limit — 108

— 110

determine instruments to carry out each test order — i

determine sequence/timing of test orders — ii

prioritize sensitive test orders — v

calculate estimated completion time — iii

— iv

cooled storage time

uncapped storage time

Effective temperature

determine lead time

N iterations

YES

lead time (of any test order) > degradation limit

No

instruct analytical instruments to carry out test orders — 112

STOP

FIG. 3

EP 3 757 575 B1

FIG. 4

22

FIG. 5

FIG. 6

FIG. 7

FIG. 8

FIG. 9

EP 3 757 575 B1

**REFERENCES CITED IN THE DESCRIPTION**

**Patent documents cited in the description**

- EP 2339353 A